# EUROPEAN PATENT APPLICATION

(11) **EP 4 151 617 A1**
(43) Date of publication of application: **22.03.2023**
(21) Application number: 21197665.9
(22) Date of filing: 20.09.2021
(51) Int. Cl.: C07C 209/84, C07C 211/12, C07C 211/09, C07C 263/10, C07C 265/14, C12P 13/00, C08G 18/00

(54) **METHOD FOR THE PROCESSING AND TRANSPORT OF HEXANE-1,6-DIAMINE OR PENTANE-1,5-DIAMINE**

(71) Applicant: Covestro Deutschland AG, 51373 Leverkusen (DE); Genomatica, Inc., San Diego CA 92121 (US)
(72) Inventor: MERKEL, Michael, 40223 Düsseldorf (DE)
(74) Representative: Scholz, Volker

(57) **Abstract**

The invention relates to a method for the processing and transport of hexane-1,6-diamine or pentane-1,5-diamine comprising the steps of:
a) Providing a first composition including 60 to 98 wt.-% water and 2 to 40 wt.-% hexane-1,6-diamine or pentane-1,5-diamine,
b) partially removing water from the first composition by distillation at a first production location, whereby a second composition is generated, said second composition including the hexane-1,6-diamine or pentane-1,5-diamine and 0.5 wt.-% to 55 wt.-% water, and
c) transporting said second composition during a span of time t from the first production location to a second production location, wherein the span of time t including times for optional temporary storage is at least 6 h.

## Description

The invention relates to a method for the processing and transport of hexane-1,6-diamine or pentane-1,5-diamine and a method for producing a polyamide or diisocyanate, in which hexane-1,6-diamine or pentane-1,5-diamine is provided as an aqueous solution at a first production location and water is partially removed, and is then transported from the first production location to a second production location and is finally further processed there into a polyamide, a diamine-dicarboxylic acid salt or a diisocyanate, optionally after further purification.

Hexane-1,6-diamine (HDA) and pentane-1,5-diamine (PDA) are important starting materials in the production of polymers. They are used for example for the production of polyamides by polycondensing the diamine with dicarboxylic acids. Polyamide-6.6 (PA66), for example, is generated from hexane-1,6-diamine and adipic acid. Another important use is the phosgenation of the diamines to the corresponding diisocyanates, which can then be converted in a further step into polyurethanes, polyureas or polyisocyanurates for example. Phosgenation places special requirements on the purity of the aliphatic diamine.

For pentane-1,5-diamine, industrial production methods have become established for some years in which the diamine is produced by biotechnological means, where first of all it is generated as a mixture with water, and the mixture is then processed into pure pentane-1,5-diamine. Recovering the amine from the aqueous fermentation medium can be carried out as described in EP2684867A1 for example, after most of the cell components have been removed. There, a method for the production of pentane-1,5-diamine is disclosed, in which the diamine is extracted in a liquid phase extraction process from the aqueous phase that has optionally been concentrated by distillation beforehand. As extractants, non-halogen aliphatic solvents, preferably straight chain alcohols with 4 to 7 carbon atoms, are recommended. These extractants must subsequently be separated from the product again by distillation. This means additional process steps and the introduction of large quantities of a further substance into the process. For the distillation processes to concentrate the aqueous raw product or to isolate the pentane-1,5-diamines from the extract, the use of a multiple column at a pressure of between 0.1 kPa and normal pressure is described in each case.

Biotechnological production processes are also known for hexane-1,6-diamine, and corresponding processes and microorganisms are disclosed in US20170369913A1 and WO2016209883A1 for example.

Isocyanates and polyamides are nowadays only produced in a small number of huge, world-scale plants, so that the amines needed for their production often have to be transported over long distances. This is especially true of aliphatic diamines produced by biotechnological methods, since the raw materials needed for this purpose are frequently not available at the locations where derivatives of the diamines are to be manufactured later.

The transport of hexane-1,6-diamine (HDA) and pentane-1,5-diamine is made even more difficult by the fact that they have melting points of approx. 40° C in the case of HDA and approx. 12° C in the case of PDA. There is therefore a risk that during the transport of these diamines there may be phase transitions from solid to liquid or *vice versa,* which are undesirable for a number of reasons. Such phase transitions may be accompanied by the release of heat and therefore pose a risk to quality or even to safety during transport. It is also a highly complex business to unload crystallised melt from a transport container or a transport ship. While it is possible to transport the diamines in heatable containers or ships, this is likewise undesirable.

It would alternatively be possible to transport the raw product and only to process it further at the location where it is needed. This approach, however, likewise involves serious disadvantages because of the large amounts to be transported and the fact that the amine content of the aqueous solutions formed is often only small.

The problem of the present invention was to provide an improved method for the processing and transport of hexane-1,6-diamine or pentane-1,5-diamine, wherein the amine is initially present in the form of a diluted aqueous solution.

This problem is solved by a method for the processing and transport of hexane-1,6-diamine or pentane-1,5-diamine comprising the steps of:
a) providing a first composition including 60 to 98 wt.-% water and 2 to 40 wt.-% hexane-1,6-diamine or pentane-1,5-diamine,
b) partially removing water from the first composition by distillation at a first production location, whereby a second composition is generated, said second composition including the hexane-1,6-diamine or pentane-1,5-diamine and 0.5 wt.-% to 55 wt.-% water, and
c) transporting said second composition during a span of time t from the first production location to a second production location, wherein the span of time t including times for optional temporary storage is at least 6 h.

In accordance with the invention, the expressions "comprising", "including" or "containing" mean preferably "consisting substantially of" and particularly preferably "consisting of".

*Statements concerning the content of organic compounds* in the context of the present invention refer, unless stated otherwise, to values determined by gas chromatography. The skilled person is familiar with the quantitative evaluation of gas chromatograms, optionally using an internal standard. The skilled person is likewise familiar with methods that might perhaps be needed to determine the *water content.* The methods known in the art can also be used in the context of the present invention. In case of doubt, the water content determined by Karl Fischer titration is decisive. The presence of amines can lead to trailing end points. In such cases, the figure determined by Karl Fischer titration after buffering with anhydrous benzoic acid is decisive. The skilled person is likewise familiar with the method using buffering with benzoic acid. For Karl Fischer titration in general, see Jander, Jahr, Massanalyse, 17th ed., de Gruyter, Berlin (2009), p. 279 to p. 282).

*"Distillation"* in the present case is understood to mean a thermal separation process used to recover evaporable substances, preferably liquids, from a composition. The separated vapours are subsequently precipitated, usually by condensation. The term encompasses in particular repeated evaporation and condensation using a column (distillation column) with a plurality of separating stages. Processes of this kind in which several distillation steps are arranged in series in a column should strictly speaking be called rectification, but will nevertheless likewise be referred to herein as distillation for the purpose of simplification. The advantage of these processes is the powerful separating effect and the possibility of operating the plant continuously. A *"distillation apparatus"* is accordingly an apparatus which is suitable for performing a thermal separation process of this kind, i.e. for example sieve plate columns, packed columns, packed-bed columns, bubble cap tray columns or even single-stage evaporators such as, for example, falling film evaporators, thin-film evaporators, flash evaporators, multi-phase helical-coil evaporators, or natural or forced circulation evaporators. The individual process steps will be explained in more detail below.

In the first step a) of the method of the invention, a first composition including 60 to 98 wt.-% water and 2 to 40 wt.-% hexane-1,6-diamine or pentane-1,5-diamine is provided.

In a further embodiment of the invention, the first composition is provided by means of recycling polyurethanes, polythiourethanes, polyureas, polyisocyanurates based on hexamethylene diisocyanate (HDI) or pentamethylene diisocyanate (PDI) and/or polyamides, especially PA64, PA66, PA69, PA610, PA612, PA54, PA56, PA59, PA510 or PA512. Recycling is preferably by hydrolysis, i.e. breaking down the polymers in the presence of water to yield monomer building blocks. Hydrolysis may be performed in the presence of catalysts, preferably as alkaline hydrolysis. The polymers used for recycling may also contain allophanate and/or biuret groups. Compositions of this kind often contain large amounts of water, which may already be present during the reaction or may for example be introduced in a subsequent extraction step. Since water can be removed from the first composition in an energy-efficient manner with the method of the invention, it is now possible to use a larger amount of water in the recycling process if required.

In a further preferred embodiment of the invention, the first composition is provided by means of the biotechnological production of hexane-1,6-diamine or pentane-1,5-diamine, for example by fermentation of suitable precursor compounds. In this case, the first composition is preferably a raw solution of the aliphatic diamine in water after any cellular components that may be present have been separated.

In a preferred embodiment of the method of the invention, the first composition includes ≥3 wt.-% and ≤35 wt.-%, preferably ≥5% wt.-% and ≤25 wt.-% and particularly preferably ≥7 wt.-% and ≤22 wt.-% hexane-1,6-diamine or pentane-1,5-diamine. In addition, the first composition usually also contains other substances apart from water which have a lower boiling point than the diamine. The content of these substances which have a lower boiling point than the amine is preferably ≤ 10 wt.-%, particularly preferably ≤ 5 wt.-% and most particularly preferably ≤ 3 wt.-%. At the same time, the content in technical processes is usually ≥ 0.1 wt.-%, preferably ≥ 0.5 wt.-%, unless low boilers have already been separated beforehand. Additionally, the first composition usually also contains other substances which have a higher boiling point than the amine. The proportion of these substances which have a higher boiling point than the amine in the first composition preferably totals ≥0.01 wt.-% and ≤10 wt.-%, particularly preferably ≥0.1 wt.-% and ≤5 wt.-%, most particularly preferably ≥0.3 wt.-% and ≤3 wt.-%.

According to the invention, the water content of the first composition is ≥60 wt.-% and ≤98 wt.-%, preferably ≥62 wt.-% and ≤95 wt.-%, particularly preferably ≥65 wt.-% and ≤90 wt.-% and most particularly preferably ≥ 75 wt.-% and ≤88 wt.-% water based on the total mass of the first composition.

Substances with lower boiling points compared to the diamine that can occur in the first composition are for example, apart from water: ammonia, alkyl or alkenyl amines, alcohols, ethylene glycol, cyclic amines such as pyrrolidine, piperidine, azepane, imines, cylic imines such as tetrahydropyridine or tetrahydroazepine, or residues of solvents such as hydrocarbons or halogenated hydrocarbons. In addition, gases such as nitrogen or carbon dioxide may be present in the composition in dissolved or bound form.

Substances with higher boiling points compared to the diamine that can occur are, for example, dimers or oligomers of the diamine, which can form while cleaving off ammonia from the diamine. In particular in the preferred embodiment - in which, as a minimum, the at least one diamine in the first composition is produced by biotechnological means -, the first composition may also contain carbohydrates which have not been completely converted in the fermentation process and salts that originate from the fermentation broth and are now still present in the first composition. If on the other hand the first composition originates from the chemical recycling of polyamides, such as PA66 or PA56, preferably by acidic hydrolysis, it may also, in the case of the polyamides mentioned by way of example, contain adipic acid, salts thereof or fragments of PA66 of PA56 as substances with high boiling points. Statements concerning relative contents refer, unless stated otherwise, to the totality of the substances with higher boiling points compared to the amine.

The partial removal of water from the first composition provided in step a) is preferably performed in a continuous distillation process. For this purpose, it is sufficient to heat the first composition and separate the vapours formed, so that the water content of the liquid composition drops and ultimately a second composition containing hexane-1,6-diamine or pentane-1,5-diamine and 0.5 wt.-% to 55 wt.-% water is obtained. With this process arrangement, however, the separated vapours still contain certain amounts of the diamine contained in the first composition. Better separation can be achieved if the distillation is performed in at least one column, with which the skilled person is familiar for such purposes and which has more than one theoretical separation stage. The at least one column preferably contains separation supporting structures to enlarge the mass transfer area. These structures may for example be different kinds of mass transfer trays (bubble cap trays, sieve trays, valve trays, etc.), packings with a random arrangement of packing material or structured packings, the latter usually involving a low pressure loss and therefore being preferable. Distillation is preferably performed at atmospheric pressure or at a reduced pressure relative to atmospheric pressure. This enables distillation at lower temperatures and reduces the risk of yield losses because of side reactions of the desired amine. Distillation is preferably performed at a pressure in the range from 0.05 to 1 bar(a), particularly preferably 0.1 to 0.8 bar(a).

In the process, water, possibly in the form of azeotropes with other compounds, is separated and removed at the head of the column as steam (vapours). The vapours are preferably condensed and the resulting condensate is preferably returned to the process from which the first composition originated, i.e. for example as fermentation medium, scrubbing liquid or as a solvent for recycling. Alternatively, the condensate may also be disposed of as effluent, optionally after further treatment to clean it.

The second composition including hexane-1,6-diamine or pentane-1,5-diamine and 0.5 wt.-% to 55 wt.-% water is obtained as a bottom product of the column. The water content is dependent on the operating conditions such as the ratio of the inlet mass flow to bottoms mass flow, the input of heating energy and the return ratio. Depending on the composition of the substances and the pressure, an evaporation temperature sets in at the bottom (bottom temperature) which can also be used to monitor the process and influence the parameters in such a way that the desired water content in the second composition is achieved.

In order to prevent the hexane-1,6-diamine or pentane-1,5-diamine from solidifying or crystallising during transport or during any temporary storage steps that may be necessary, the removal of water does not continue until the composition is dry, but takes the form of a partial removal of water, so that a second composition is obtained which has a water content of 0.5 wt.-% to 55 wt.-%. A larger proportion of water provides greater stability against undesirable phase transitions. At the same time however, the water content should not be adjusted to too high a level in order not to render the transport inefficient. In a further preferred embodiment, the water content of the second composition is therefore in the range from 1 wt.-% to 35 wt.-%, preferably in the range from 2 wt.-% to 27 wt.-% and particularly preferably in the range from 5 wt.-% to 20 wt.-% water based on the entire second composition.

Since the second composition in the method of the invention is formed as a bottom product of distilllation, it usually contains small amounts of dimerisation products of the general formula (I) or (II):

H₂N-[CH₂]ₙ-NH-[CH₂]ₙ-NH₂ (I)

H₂N-[CH₂]ₙ-N=CH-[CH₂]ₙ₋₁-NH₂ (II)

wherein n=5 or 6.

Dimers of this kind are used for example in order to incorporate functionalities into diamine-based polymers and to modify their properties.

A further subject-matter of the invention is therefore second compositions including, based on the total mass of the second composition, 0.5 wt.-% to 55 wt.-%, preferably 1 wt.-% to 35 wt.-% and particularly preferably 2 wt.-% to 27 wt.-% water, 45 wt.-% to 99 wt.-%, preferably 65 wt.-% to 99 wt.-% of an aliphatic diamine and in total 0.0005 wt.-% to 5 wt.-%, preferably 0.001 wt.-% to 3 wt.-% and particularly preferably 0.01 wt.-% to 2 wt.-% of dimeric compounds according to formula (I) and formula (II).

The second composition, which is formed in the course of distillation, is transported, preferably in liquid form, from the first production location to a second production location over a span of time t. The transport is preferably carried out in vessels selected from the group consisting of transport containers, tank waggons or tankers. Transport in tankers is particularly preferable. The span of time t includes not only the actual transport, i.e. the process of shipping the second composition from one production location to the other production location, but also any temporary storage in tanks that may be necessary after the removal of water at the first production location and before phosgenation or polycondensation and any further purification that may be necessary at the second production location. It thus corresponds to the span of time from when the second composition is filled into a storage tank or transport container after it has been removed from the distillation apparatus until the time when the second composition is fed from a storage tank or transport container into a process for further use to be made of the second composition at the second production location. According to the invention, the span of time t is at least 6 h. The longer the span of time, the greater the benefits that can be reaped from the method of the invention. This is why in a further preferred embodiment the span of time t is at least 24 h, preferably at least 168 h and particularly preferably at least 336 h.

A further advantage of the method of the invention is the fact that aqueous compositions of the diamine are handled both at the first and at the second production location. Thanks to the good solubility of hexane-1,6-diamine and pentane-1,5-diamine in water, it is consequently possible to rinse transport containers or temporary storage tanks with water if necessary and to blend the rinsing water enriched with the diamine concerned in the process. Complex disposal of rinsing water or the use of other rinsing agents is no longer necessary.

The second composition, from which part of the water has been removed and which has been transported to the second production location, is particularly suitable for the production of aliphatic diisocyanates, polyamides or diamine-dicarboxylic acid salts. It is therefore also preferable for the method of the invention to comprise the following steps after step c):
d) optionally further purifying the second composition at the second production location, thus generating a third composition,
   and either
e) phosgenating the second and/or the optionally obtained third composition at the second production location so that at least one aliphatic diisocyanate is generated
   or
f) polycondensing the second and/or the optionally obtained third composition at the second production location so that a polyamide is generated
   or
g) converting the second and/or the optionally obtained third composition with a dicarboxylic acid to the diamine-dicarboxylic acid salt.

A further subject-matter of the invention is therefore a method for producing a polyamide or an aliphatic diisocyanate comprising the steps of:
a) providing a first composition including 60 to 98 wt.-% water and 2 to 40 wt.-% hexane-1,6-diamine or pentane-1,5-diamine,
b) partially removing water from said first composition by distillation at a first production location, whereby a second composition is generated, said second composition including the hexane-1,6-diamine or pentane-1,5-diamine and 0.5 wt.-% to 55 wt.-% water, and
c) transporting said second composition during a span of time t from the first production location to a second production location, wherein the span of time t including times for optional temporary storage is at least 6 h.
d) optionally further purifying the second composition at the second production location, thus generating a third composition,
   and either
e) phosgenating the second and/or the optionally obtained third composition at the second production location so that at least one aliphatic diisocyanate is generated
   or
f) polycondensing the second and/or the optionally obtained third composition at the second production location so that a polyamide is generated
   or
g) converting the second and/or the optionally obtained third composition with a dicarboxylic acid to the diamine-dicarboxylic acid salt.

After transport to the second production location and any temporary storage at the second production location that may be necessary, the second composition may optionally be subjected to further purification so that a third composition is generated. This is preferably done by distillation or a combination of distillation and extraction steps. The skilled person is sufficiently familiar with processes for doing this.

The purification of diamines by distillation is usually performed in, for example, a distillation sequence involving several steps. First of all, water and certain low boilers, especially those which form an azeotrope with water, are separated in a dewatering column over the head. Depending on the configuration of the water removal process in step b) of the method of the invention, this step might be dispensed with if it has been carried out sufficiently at the first production location. Especially if any residual amounts of water remaining do not disturb the further conversion, for example because this takes place in the aqueous medium anyway, this first step may perhaps be dispensed with in the process of purification by distillation. After that, high and low boilers are separated, with the steps performed either in individual distillation columns or combined in a dividing wall column. If two separate distillation columns are used, it is preferable first to remove the high boilers from a distillation column as a bottom product and only then to withdraw the further low boilers over the head of a further distillation column. Finally, it is preferable to perform a fine distillation step yielding the third composition.

If the diamine contained in the second composition is to be subjected in step e) to conversion with phosgene to the corresponding diisocyanate, it is advantageous to lower the water content of the composition in step d) to ≤0.2 wt.-%, preferably ≤0.1 wt.-% and particularly preferably to ≤0.05 wt.-% in order to decrease the formation of hydrochloric acid in the phosgenation process and to reduce the associated corrosion problems.

Especially when the second composition has a higher water content, i.e. for example at water contents ≥20 wt.-%, preferably ≥30 wt.-%, it may be advantageous first to extract the amine from the second composition by extraction with an organic solvent and then to process the extract further by distillation. Because of the lower evaporation enthalpy of most organic solvents compared to water, this results in a reduced energy consumption for the process compared to processing purely by distillation.

The second composition and/or the optionally obtained third composition can be converted with phosgene in step e) of the method of the invention to yield at least one aliphatic diisocyanate.

Alternatively, it can be converted in a polycondensation reaction with a dicarboxylic acid or a dicarboxylic acid derivative in step f) of the method of the invention so that a polyamide is generated. Since the second or third composition is substantially hexane-1,6-diamine or pentane-1,5-diamine, the expression "aliphatic diamine" will be used instead of the expressions "second composition" and "third composition" in the following description of the phosgenation reaction and polycondensation in order to render the text more easily readable.

The aliphatic diamine can be converted into a polyamide in step f) in a polycondensation reaction with a dicarboxylic acid and/or a dicarboxylic acid derivative selected from the group consisting of dicarboxylic acid diesters, dicarboxylic acid dichlorides and dicarboxylic acid anhydrides. It is preferable for the polycondensation to be performed with a dicarboxylic acid with elimination of water. In this embodiment, the aliphatic diamine is heated in an aqueous solution together with a dicarboxylic acid and is thus converted into the polyamide in a polycondensation reaction. Optional intermediate steps may be performed, such as the production and isolation of the salt from the aliphatic diamine and the dicarboxylic acid for example. The conversion is preferably performed under pressure, with the pressure being controlled by deliberately releasing surplus water vapour. In order to build up high molecular weights, the pressure is reduced in the further course of the reaction and the water is preferably removed from the reaction mixture completely.

A further alternative is to convert the aliphatic diamine with a dicarboxylic acid to the diamine-dicarboxylic acid salt in step g). The conversion is preferably performed in an aqueous solution, with the equivalence of the components preferably being adjusted via the pH. If it is to be expected that diamine losses will occur in a later polymerisation of the AH salt, for example, a slight stoichiometric excess of 0.5% to 5%, preferably 1% to 3% of the diamine relative to the carboxylic acid can be used in the production of the diamine-dicarboxylic acid salt, so that the pH is more than 7.0. The salt can be crystallised by evaporating water and/or cooling the hot solution. If necessary, it can be purified by recrystallisation, for example from methanol.

The phosgenation of the aliphatic diamine in step e) may for example be performed in the gaseous phase. The skilled person is sufficiently familiar with methods for the gas phase phosgenation of aliphatic diamines known in the art. Phosgenation is performed at temperatures ranging from 200 to 600° C with an excess of phosgene, optionally in the presence of an inert gas or vapours of an inert solvent.

After conversion, which is performed in a preferably cylindrical reaction space, the diisocyanate formed is removed from the reaction mixture, preferably by selective condensation in an inert solvent such as chlorobenzene or dichlorobenzene, and is then processed into pure diisocyanate in a multiple distillation process.

In a further embodiment, the phosgenation of the aliphatic diamine in step e) takes place in the liquid phase. The reaction can then be performed in various ways. Either the aliphatic diamine is converted directly with an excess of phosgene in an inert liquid medium, preferably in a two-stage process known as cold-hot phosgenation (base phosgenation), or the corresponding salt is first transformed by conversion with hydrogen chloride gas or carbon dioxide in an inert liquid medium and then converted with an excess of phosgene similar to the hot phosgenation step in base phosgenation (hydrochloride or carbaminate phosgenation). A suitable liquid medium for all phosgenations is in particular chlorobenzene and/or dichlorobenzene. The skilled person is also familiar with these processes for phosgenation in the liquid phase.

Both in base phosgenation and in amine-hydrochloride or carbaminate phosgenation, the remaining phosgene and hydrogen chloride gas is preferably blown off with an inert gas, preferably nitrogen, after the reaction is completed. If needed, filtration may be performed in order to remove any solids that might be present, such as unreacted amine-hydrochlorides. The hexane-1,6-diamine or pentane-1,5-diisocyanate formed is then preferably processed to the pure diisocyanate by multiple distillation.

### Examples

### Example 1 (comparative example, not in accordance with the invention):

From an aqueous solution containing 20 wt.-% pentane-1,5-diamine, water was distilled off over the head in a distillation apparatus at 500 mbar(a). In the bottom of the distillation apparatus, a pentane-1,5-diamine remained with a water content of approx. 0.1 %. A sample of this bottom product was stored for 24 h at 5° C in order to reproduce the conditions of transport, during which time crystallisation occurred.

### Example 2 (in accordance with the invention):

From an aqueous solution containing 20 wt.-% pentane-1,5-diamine, water was distilled off over the head in a distillation apparatus at 500 mbar(a). Distillation was stopped when there was a water content of approx. 13 wt.-% in the bottom. A sample of this bottom product was stored at 5° C in order to reproduce the conditions of transport. It remained liquid even after a week.

The remaining bottom product was then fractionated and distilled further, and a fraction of pentane-1,5-diamine with a purity of 99.95% was obtained. This was subjected to base phosgenation in the form of cold-hot phosgenation in a laboratory apparatus and, as expected, pentane-1,5-diisocyanate was obtained after processing.

### Example 3 (comparative example, not in accordance with the invention):

From an aqueous solution containing 20 wt.-% hexane-1,6-diamine, water was distilled off over the head in a distillation apparatus at 500 mbar(a). In the bottom of the distillation apparatus, a hexane-1,6-diamine remained with a water content of approx. 0.1 %. A sample of this bottom product was stored for 24 h at 20° C in order to reproduce the conditions of transport, during which time crystallisation occurred.

### Example 4 (in accordance with the invention):

From an aqueous solution containing 20 wt.-% hexane-1,6-diamine, water was distilled off over the head in a distillation apparatus at 500 mbar(a). Distillation was stopped when there was a water content of approx. 20 wt.-% in the bottom. A sample of this bottom product was stored at 20° C in order to reproduce the conditions of transport. It remained liquid even after a week.

A partial amount of the bottom product was heated to 60° C and converted to the AH salt with adipic acid, working with a slight excess of HDA, in order to adjust it to a pH of 7.1. The reaction mixture was boiled down in a vacuum and the solid precipitating in the process was filtered off. An 80% solution of this solid in water was first subjected to precondensation in an autoclave at a constant pressure of 17 bar(a) and a temperature of 270° C before polycondensation to PA66 was completed at a pressure dropping down to 1 bar(a), and thus with the complete removal of the water.

### Example 5:

By way of example, the setting points of various mixtures consisting of hexane-1,6-diamine and water or pentane-1,5-diamine and water depending on the water content were determined. The results are summed up in the following Table. The results, taking the transport and storage conditions into account, give the skilled person an indication of the residual water content that must be present in the second composition in order reliably to rule out phase transitions from liquid to solid during the transport or temporary storage of the second composition.

**Table 1: Survey of the solidification temperatures of hexane-1,6-diamine/water and pentane-1,5-diamine/water mixtures**

| Water content [wt.-%] | 0.0 | 1.0 | 3.0 | 5.0 | 10.0 | 15.0 | 20.0 | 27.0 | 33.0 | 38.0 |
|---|---|---|---|---|---|---|---|---|---|---|
| Solidification temperature hexane-1,6-diamine/water [°C] | 40.5 | 39.4 | 37.2 | 35.0 | 29.5 | 23.8 | 17.8 | 9.5 | 2.5 | -3.0 |
| Solidification temperature pentane-1,5-diamine/water [°C] | 15.1 | 14.0 | 11.7 | 9.7 | 4.6 | -2.6 | -12.2 | -29.2 | -38.2 | -43.9 |

## Claims

1. Method for the processing and transport of hexane-1,6-diamine or pentane-1,5-diamine comprising the steps of:
a) providing a first composition including 60 to 98 wt.-% water and 2 to 40 wt.-% hexane-1,6-diamine or pentane-1,5-diamine,
b) partially removing water from said first composition by distillation at a first production location, whereby a second composition is generated, said second composition including the hexane-1,6-diamine or pentane-1,5-diamine and 0.5 wt.-% to 55 wt.-% water, and
c) transporting said second composition during a span of time t from the first production location to a second production location, wherein the span of time t including times for optionally temporary storage is at least 6 h.

2. The method according to claim 1, **characterised in that** the first composition includes 2 to 40 wt.-% hexane-1,6-diamine.

3. The method according to claim 1, **characterised in that** the first composition includes 2 to 40 wt.-% pentane-1,5-diamine.

4. The method according to any one of claims 1 to 3, **characterised in that** the first composition provided in step a) is obtained by recycling polyurethanes, polythiourethanes, polyureas, polyisocyanurates and/or polyamides.

5. The method according to any one of claims 1 to 3, **characterised in that** the first composition provided in step a) is obtained by biotechnological production of the diamine, for example by fermentation of suitable precursor compounds.

6. The method according to any one of claims 1 to 5, **characterised in that** the first composition includes ≥3 wt.-% and ≤35 wt.-%, preferably ≥5% wt.-% and ≤25 wt.-% and particularly preferably ≥7 wt.-% and ≤22 wt.-% hexane-1,6-diamine or pentane-1,5-diamine.

7. The method according to any one of claims 1 to 6, **characterised in that** the water content of the first composition is ≥62 wt.-% and ≤95 wt.-%, preferably ≥65 wt.-% and ≤90 wt.-% and particularly preferably ≥75 and ≤88 wt.-%.

8. The method according to any one of claims 1 to 7, **characterised in that** the water content of the second composition is in the range from 1 wt.-% to 35 wt.-%, preferably in the range from 2 wt.-% to 27 wt.-% and particularly preferably in the range from 5 wt.-% to 20 wt.-% water based on the entire second composition.

9. The method according to any one of claims 1 to 8, **characterised in that** the transport takes place in vessels selected from the group consisting of transport containers, tank waggons and tankers.

10. The method according to any one of claims 1 to 9, **characterised in that** the span of time t is at least 24 h, preferably at least 168 h and particularly preferably at least 336 h.

11. Method for producing a polyamide or an aliphatic diisocyanate comprising the steps of:
a) providing a first composition including 60 to 98 wt.-% water and 2 to 40 wt.-% hexane-1,6-diamine or pentane-1,5-diamine,
b) partially removing water from said first composition by distillation at a first production location, whereby a second composition is generated, said second composition including the hexane-1,6-diamine or pentane-1,5-diamine and 0.5 wt.-% to 55 wt.-% water, and
c) transporting said second composition during a span of time t from the first production location to a second production location, wherein the span of time t including times for optional temporary storage is at least 6 h.
d) optionally further purifying the second composition at the second production location, thus generating a third composition
and either
e) phosgenating the second and/or the optionally obtained third composition at the second production location so that hexane-1,6-diamine or pentane-1,5-diisocyanate is generated;
or
f) polycondensing the second and/or the optionally obtained third composition at the second production location so that a polyamide based on hexane-1,6-diamine or pentane-1,5-diamine is generated
or
g) converting the second and/or the optionally obtained third composition with a dicarboxylic acid to the diamine-dicarboxylic acid salt.

12. A second composition including based its total mass 0.5 wt.-% to 55 wt.-%, preferably 1 wt.-% to 35 wt.-% and particularly preferably 2 wt.-% to 27 wt.-% water, 45 wt.-% to 99 wt.-%, preferably 65 wt.-% to 99 wt.-% of an aliphatic diamine and in total 0.0005 wt.-% to 5 wt.-%, preferably 0.001 wt.-% to 3 wt.-% and particularly preferably 0.01 wt.-% to 2 wt.-% of dimeric compounds according to formula (I) and formula (II).
H₂N-[CH₂]ₙ-NH-[CH₂]ₙ-NH₂ (I)
H₂N-[CH₂]ₙ-N=CH-[CH₂]ₙ₋₁-NH₂ (II)
wherein n = 5 or 6.
